# FASCICULE DE BREVET EUROPEEN

(11) **EP 3 367 937 B1**
(45) Date de publication et mention de la délivrance du brevet: **31.07.2019**
(21) Numéro de dépôt: 16787856.0
(22) Date de dépôt: 27.10.2016
(51) Int. Cl.: A61B 17/435

(54) **DISPOSITIF INTRA-UTÉRIN RÉCUPÉRABLE**
RÜCKHOLBARE INTRAUTERINVORRICHTUNG
RETRIEVABLE INTRAUTERINE DEVICE

(30) Priorité: 30.10.2015 FR 1560439
(43) Date de publication de la demande: 05.09.2018
(73) Titulaire: Anecova S.A., 1015 Lausanne (CH)
(72) Inventeur: JOIE, Michel, 5030 Ernage (BE); RIVIERE, Stéphane, 01170 Segny (FR); GROGG, Julien, 1844 Villeneuve (CH)
(74) Mandataire: Santarelli
(86) Numéro de dépôt international: PCT/EP2016/075877
(87) Numéro de publication internationale: WO 2017/072204

(56) Documents cités:
- EP-A1- 2 057 972
- WO-A2-03/011200
- FR-A1- 2 895 229
- US-A- 5 135 865
- US-A- 5 505 716
- US-A1- 2006 228 794
- C. BLOCKEEL ET AL: "An in vivo culture system for human embryos using an encapsulation technology: a pilot study", HUMAN REPRODUCTION, vol. 24, no. 4, 26 décembre 2008 (2008-12-26), pages 790-796, XP055286577, GB ISSN: 0268-1161, DOI: 10.1093/humrep/dep005
- R. FRYDMAN ET AL: "INVO: a simple, low cost effective assisted reproductive technology", ESHRE MONOGRAPHS, vol. 2008, no. 1, 1 juillet 2008 (2008-07-01), pages 85-89, XP055286476, ISSN: 1477-741X, DOI: 10.1093/humrep/den163

## Description

La présente invention concerne un dispositif intra-utérin récupérable, utilisé notamment pour placer temporairement des gamètes et/ou des embryons dans l'utérus.

La présente invention concerne de manière générale le domaine de la fécondation in-vivo et in-utéro dans les méthodes de reproduction assistées avec mise en oeuvre des techniques de fertilisation et/ou de développement préimplantatoire.

Les systèmes intra-utérins implantables et récupérables par les voies naturelles sont connus depuis plusieurs années.

On connait ainsi un dispositif intra-utérin tel que décrit dans le document WO 03/011200 utilisé dans l'assistance in-vivo dans les processus de procréation médicalement assistés.

Un tel dispositif est destiné à être placé dans la cavité utérine pour une période comprise entre quelques heures et quelques jours et permet le développement préimplantatoire d'un embryon in-vivo.

Le dispositif intra-utérin comporte un logement adapté à contenir les éléments destinés à être encapsulés dans le dispositif, et par exemple un embryon, des gamètes males et/ou femelles, un ovocyte fécondé, un ovule non fécondé ou encore une combinaison de ces éléments.

Cette technique d'encapsulation permet de réaliser la fécondation et le développement d'un embryon à l'intérieur d'une capsule placée dans l'utérus.

La Demanderesse a constaté qu'en plaçant transitoirement la capsule avec un embryon en cours de développement dans l'utérus, les embryons ainsi obtenus étaient de meilleure qualité non seulement sur le plan morphologique mais aussi sur leur capacité à se développer et à être réimplantés dans l'utérus ultérieurement.

Ainsi, en obtenant des embryons de meilleure qualité, il est possible de diminuer les occurrences de grossesses multiples en autorisant la réimplantation d'un unique embryon avec des chances de succès supérieures à celles obtenues dans les méthodes classiques de reproduction assistée in-vitro.

Par ailleurs, la fécondation et le développement préimplantatoire ayant lieu directement dans l'utérus, les répercussions psychologiques sur le couple peuvent être importantes dès lors que le procédé de reproduction assistée est plus proche de la conception naturelle et requiert une implication supérieure du couple.

Le dispositif intra-utérin permet de placer les embryons dans un environnement naturel pendant la phase de développement préimplantatoire, et autorise ainsi les interactions complexes entre l'endomètre de l'utérus et les gamètes et/ou les embryons.

En minimisant l'impact des conditions de culture artificielles (par exemple, des milieux de culture de synthèse) sur le développement de l'embryon, les risques d'altération de l'embryon par des facteurs étrangers ou par l'absence de molécules importantes pour la régulation du développement sont diminués.

Dans le document WO 03/011200, le dispositif intra-utérin récupérable comporte un logement dont la paroi est réalisée à partir d'une membrane perméable poreuse, et par exemple en polyéthersulfone (PES).

Le choix d'une telle membrane poreuse a été guidé initialement pour permettre la protection des gamètes et/ou embryons placés dans le dispositif tout en permettant le passage de nutriments, présents dans le milieu utérin, à l'intérieur du logement du dispositif.

Toutefois, une membrane perméable poreuse est fragile et difficile à manipuler, notamment lors du chargement et déchargement des éléments encapsulés, ou encore lors de l'implantation et de la récupération de la capsule à l'intérieur de l'utérus.

Il est primordial en effet de ne pas détériorer la paroi de la capsule, ce qui pourrait provoquer la perte des éléments encapsulés.

On connait également dans le document FR 2 895 229 un dispositif intra-utérin récupérable comprenant un logement ayant une paroi dans laquelle sont réalisées des perforations dont les dimensions sont comprises entre 10 µm et 150 µm.

Toutefois, la taille importante de ces perforations peut parfois présenter un caractère délétère en autorisant le passage de cellules néfastes au développement de l'embryon.

Le document US2006228794 A1 décrit un récipient comprenant un réceptacle destiné à contenir un milieu biologique, des gamètes et/ou un ou plusieurs embryons. La cuve est munie d'un joint perméable au CO₂ et d'un dispositif de fermeture pour un accès sélectif. Une chambre tampon avec une atmosphère enrichie en CO₂ entoure au moins partiellement la cuve et est définie par une enveloppe. La chambre tampon est en communication avec la paroi perméable au CO₂. Le joint perméable empêche la pénétration des sécrétions vaginales.

La présente invention a pour but de résoudre au moins certains des inconvénients précités et de proposer un dispositif intra-utérin récupérable favorisant les échanges avec la matrice utérine, de manière fiable et reproductible, et de manipulation aisée, sans risque d'altération de la paroi du logement.

A cet effet, le dispositif intra-utérin récupérable selon l'invention comprend un logement, ledit logement comprenant une paroi interne réalisée en un polymère biocompatible, ladite paroi interne comprenant au moins une partie perforée ayant des perforations de dimensions comprises entre 1 µm et 10 µm, et une paroi externe recouvrant ladite paroi interne, la paroi externe étant réalisée en un élastomère de silicone biocompatible et comprenant au moins une ouverture en vis-à-vis de ladite au moins une partie perforée de la paroi interne.

Ainsi, le logement comprend une double paroi. La partie perforée de la paroi interne forme une frontière entre l'intérieur du logement, destiné à contenir des gamètes ou un embryon, et la matrice utérine dans laquelle est placé le dispositif intra-utérin. La paroi interne autorise ainsi les échanges intra-utérins grâce aux perforations.

Par ailleurs, la paroi externe du logement facilite la manipulation du logement par le biologiste et/ou le médecin, en formant une protection en un élastomère de silicone.

La paroi externe permet ainsi de manipuler le logement sans risque de détérioration ou rupture de la paroi interne, plus fragile, comportant la partie perforée pour favoriser les échanges avec la matrice utérine.

La paroi interne du dispositif intra-utérin récupérable présente les caractéristiques suivantes, seules ou en combinaison les unes avec les autres :
- les perforations ont des dimensions comprises entre 1 µm et 10 µm, et de préférence entre 2 µm et 8 µm ;
- la paroi interne est réalisée en polycarbonate ;
- la paroi interne a au moins une partie perforée comprenant de 1 million à 5 millions de perforations par cm² ;
- les perforations ont sensiblement une forme circulaire de diamètre compris entre 1 µm et 10 µm, et de préférence entre 2 µm et 8 µm.

Dans un mode de réalisation, le logement est de forme allongée selon une direction longitudinale, la paroi externe s'étendant au-delà des extrémités de la paroi interne dans la direction longitudinale.

La protection formée par la paroi externe s'étend ainsi sur toute la longueur du logement, et au-delà de la paroi interne.

Avantageusement, le dispositif comporte au moins un bouchon monté à une extrémité du logement afin d'obturer celui-ci lors de son utilisation.

Dans un mode de réalisation, le bouchon est monté par friction à une des extrémités de la paroi interne du logement et est partiellement recouvert par une extrémité de la paroi externe.

Un tel montage assure une bonne étanchéité du système de fermeture du bouchon.

Selon un exemple de réalisation, le logement est de forme cylindrique allongée.

La paroi externe comprend au moins une ouverture s'étendant dans la direction longitudinale du logement et sur un secteur angulaire du logement compris entre 80° et 120°.

En pratique, la paroi interne comprend deux parties perforées, diamétralement opposées dans la forme cylindrique allongée du logement, et la paroi externe comprend deux ouvertures en vis-à-vis respectivement des deux parties perforées de la paroi interne.

La configuration du logement résulte d'un compromis entre une surface importante d'échange avec la matrice utérine, au niveau des parties perforées de la paroi interne, placées en vis-à-vis des ouvertures de la paroi externe, et une bonne tenue et manipulation du dispositif intra-utérin, grâce aux portions de paroi externe.

De préférence, la paroi interne et la paroi externe sont réalisées en matériau transparent.

L'utilisation d'un matériau transparent permet de faciliter la manipulation du dispositif, notamment lors du chargement et du déchargement du dispositif, grâce à la visualisation des gamètes ou des embryons dans le logement.

Il est en outre possible d'observer de l'extérieur du dispositif le développement des ovocytes et/ou des embryons encapsulés à l'intérieur du dispositif.

Selon un autre aspect, la présente divulgation concerne également l'utilisation du dispositif intra-utérin pour charger et/ou décharger des éléments choisis parmi le groupe comprenant un embryon, des gamètes males et/ou femelles, un ovocyte fécondé, un ovule non fécondé et une combinaison de ces éléments.

D'autres particularités et avantages de l'invention apparaîtront encore dans la description ci-après.

Aux dessins annexés, donnés à titre d'exemples non limitatifs :
- la figure 1 est une vue en perspective éclatée partielle d'un dispositif intra-utérin récupérable selon un mode de réalisation de l'invention ;
- la figure 2 est une vue en coupe longitudinale selon la ligne II-II du dispositif intra-utérin récupérable de la figure 1 ;
- la figure 3 est une vue en coupe transversale selon la ligne III-III à la figure 1 ; et
- la figure 4 est une vue en coupe illustrant schématiquement le dispositif intra-utérin récupérable selon un mode de réalisation placé dans une cavité utérine.

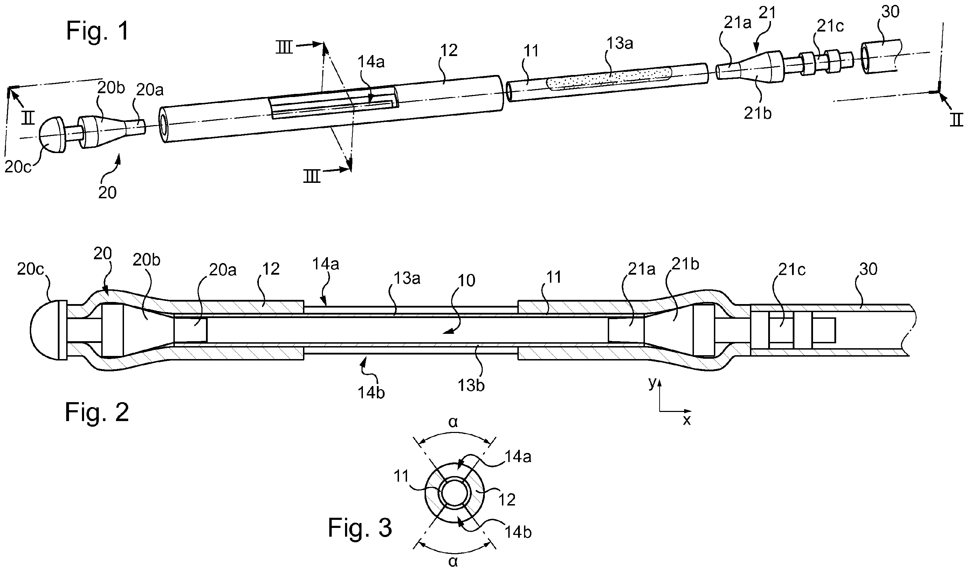

On va décrire tout d'abord en référence aux figures 1 à 3 un exemple de réalisation d'un dispositif intra-utérin récupérable.

De manière générale, le dispositif intra-utérin récupérable comprend un logement 10 destiné à contenir différents éléments mis en oeuvre lors d'un processus de procréation médicalement assistée.

En particulier, le logement 10 est adapté à contenir des éléments variés en fonction de l'état d'avancement du processus de fécondation, et par exemple des gamètes males et/ou des gamètes femelles, un ovule non fécondé, ou un ovocyte fécondé, ou un ou plusieurs embryons.

On notera que le dispositif intra-utérin récupérable est adapté à contenir l'un ou l'autre de ces éléments en fonction de l'état d'avancement dans le temps du processus de fécondation.

Le logement 10 est particulier en ce qu'il comprend une double paroi formée d'une paroi interne 11 et d'une paroi externe 12.

La paroi interne 11 est réalisée en un polymère biocompatible.

La paroi interne 11 étant destinée à former la cavité intérieure du logement 10, il est important que le matériau utilisé soit de qualité médicale et ne crée pas de conditions néfastes au développement des embryons.

A titre d'exemple, la paroi interne 11 peut être réalisée en polycarbonate.

Bien entendu, d'autres types de polymère biocompatible pourraient être utilisés, et par exemple un polyimide.

Afin d'autoriser les échanges avec le milieu intra-utérin, la paroi interne 11 comprend au moins une partie perforée 13a, 13b.

Dans ce mode de réalisation, et de manière non limitative, le logement est de forme cylindrique allongée selon une direction longitudinale X.

Ainsi, la paroi interne 11 du logement 10 présente également une forme cylindrique allongée.

Dans ce mode de réalisation, comme bien illustré à la figure 2, la paroi interne 11 comprend deux parties perforées 13a, 13b diamétralement opposées dans la forme cylindrique allongée de la paroi interne 11.

Bien entendu, ce mode de réalisation n'est nullement limitatif, et la paroi interne pourrait comporter une unique partie perforée ou au contraire, plus de deux parties perforées.

La Demanderesse a constaté que les perforations devaient présenter des dimensions suffisantes pour favoriser les échanges avec les nutriments présents dans le milieu utérin, mais sans atteindre des dimensions telles que les éléments contenus dans le logement 10 puissent s'échapper dans la matrice utérine de manière incontrôlée ou que des cellules délétères puissent pénétrer à l'intérieur du logement 10.

A cet effet, les perforations ont des dimensions comprises entre 1 µm et 10 µm.

Les perforations peuvent bien entendu avoir des formes diverses.

Quelle que soit sa forme, une telle perforation aura des dimensions comprises entre 1 µm et 10 µm dès lors qu'un cercle circonscrit à cette perforation présente un diamètre compris entre 1 µm et 10 µm.

Plus particulièrement, des perforations ayant des dimensions comprises entre 2 µm et 8 µm sont privilégiées pour les applications envisagées.

Comme cela apparaitra ensuite dans la description d'un procédé de réalisation d'un tel logement, les perforations peuvent avoir sensiblement une forme circulaire de diamètre compris entre 1 µm et 10 µm, et préférentiellement entre 2 µm et 8 µm.

Les perforations sont de préférence disposées de manière aléatoire et homogène dans la partie perforée 13a, 13b.

La densité de ces perforations doit être suffisante dans la partie perforée 13a, 13b pour créer une surface d'échange adéquate avec le milieu intra-utérin.

A titre d'exemple, la partie perforée 13a, 13b comprend entre un million à cinq millions de perforations par cm².

La paroi externe 12 est réalisée en un élastomère de silicone biocompatible.

Tout type de silicone (ou siloxane polymérisé) se présentant sous la forme d'un élastomère peut être utilisé.

D'autres types d'élastomère biocompatible pourraient être utilisés, par exemple un thermoplastique élastomère de polyurethane ou un elastomère de type EVC obtenu par un procédé de vulcanisation à chaud (EVC : acronyme d'Elastomère Vulcanisable à Chaud).

La paroi externe 12 comprend au moins une ouverture 14a, 14b destinée à être positionnée en vis-à-vis d'une partie perforée 13a, 13b de la paroi interne 11 lorsque la paroi externe 12 recouvre la paroi interne 11.

Dans ce mode de réalisation, le logement 10 étant de forme cylindrique allongée, la paroi externe 12 a également une forme générale de cylindre allongé.

De manière non limitative, dans ce mode de réalisation où la paroi interne comprend deux parties perforées 13a, 13b, la paroi externe 12 comprend deux ouvertures 14a, 14b en vis-à-vis respectivement des deux parties perforées 13a, 13b de la paroi interne 11.

Bien entendu, le nombre et la disposition des ouvertures 14a, 14b dans la paroi externe 12 sont liés intrinsèquement au nombre et à la disposition des parties perforées 13a, 13b de la paroi interne 11.

Dans le mode de réalisation décrit en référence aux figures 1 à 3, la paroi externe 12 comprend deux ouvertures 14a, 14b qui s'étendent chacune dans la direction longitudinale X du logement 10.

Comme bien illustré à la figure 3, les deux ouvertures 14a, 14b sont diamétralement opposées dans la forme cylindrique allongée de la paroi externe 12.

Chaque ouverture 14a, 14b s'étend sur un secteur angulaire α du logement 10 compris entre 80° et 120°.

De préférence, lorsque la paroi externe 12 comprend deux ouvertures 14a, 14b, le secteur angulaire α de chacune des ouvertures est compris entre 80° et 90°.

Dans ce mode de réalisation, on a illustré à titre d'exemple non limitatif deux ouvertures 14a, 14b présentant un secteur angulaire α de même valeur.

Bien entendu, l'invention n'est pas limitée à ce mode de réalisation et les ouvertures de la paroi externe 12 peuvent présenter des secteurs angulaires de valeurs différentes.

En particulier, on peut privilégier un mode de réalisation dans lequel la somme des secteurs angulaires α des ouvertures prévues dans la paroi externe 12 reste inférieur à 180° de telle sorte que la paroi externe 12 puisse conserver une rigidité suffisante dans la longueur du logement 10, notamment pour permettre le maintien du système de fermeture.

De même, la longueur selon la direction longitudinale X de chaque ouverture 14a, 14b est comprise entre un quart et la moitié de la longueur de la paroi externe 12 du logement 10, et de préférence sensiblement égale à un tiers de cette longueur.

De préférence, le polymère biocompatible utilisé pour réaliser la paroi interne 11 et l'élastomère de silicone biocompatible utilisé pour réaliser la paroi externe 12 sont des matériaux transparents, permettant au biologiste et/ou au médecin d'observer l'intérieur de la capsule lors de sa manipulation.

On va donner de manière non limitative un exemple dimensionnel d'un logement 10 du dispositif intra-utérin récupérable.

La paroi interne 11 peut présenter un diamètre intérieur de 350 µm et un diamètre extérieur de 500 µm.

L'épaisseur de la paroi interne 11 est ainsi sensiblement égale à 100 µm.

La longueur selon la direction longitudinale X de la paroi interne 11 est comprise entre 4 mm et 6 mm de long.

En correspondance, la paroi externe 12 possède un diamètre intérieur compris entre 300 µm et 500 µm et de préférence égale à 430 µm.

Le diamètre extérieur de la paroi externe 12 est compris entre 700 µm et 800 µm et de préférence égale à 800 µm.

L'épaisseur de la paroi externe 12 est ainsi comprise entre 200 µm et 400 µm.

La longueur selon la direction longitudinale X de la paroi externe 12 est comprise entre 7 mm et 8 mm.

Les fenêtres 14a, 14b s'étendent ainsi selon la direction longitudinale X sur une longueur comprise entre 2 mm et 3 mm, et de préférence présentent une longueur égale à 2,8 mm.

Le secteur angulaire de chaque ouverture 14a, 14b est sensiblement égal à 80°.

Indépendamment des dimensions spécifiques indiquées ci-dessus, le logement se présente sous une forme allongée selon la direction longitudinale X, la paroi externe 12 s'étendant au-delà des extrémités de la paroi interne 11 dans la direction longitudinale X.

On obtient ainsi un logement 10 avec une paroi externe 12 formant cage de protection autour de la paroi interne 11.

On va décrire à présent à titre d'exemple non limitatif un procédé de réalisation d'un tel logement 10 à double-paroi.

On met en oeuvre un tube cylindrique en élastomère de silicone coupé à longueur pour former la paroi externe 12.

Les ouvertures 14a, 14b peuvent être réalisées par exemple par découpe laser ou encore par découpe au moyen d'un jet d'eau sous pression.

Des perforations sont réalisées sur une portion tubulaire de polymère, tel que du polycarbonate pour constituer la paroi interne 11.

La paroi interne 11 est coupée à longueur, puis introduite dans la paroi externe 12.

Les parties perforées de la paroi interne 11 apparaissent à nu au travers des ouvertures 14a, 14b de la paroi externe 12.

A cet effet, on peut mettre en oeuvre par exemple une technologie dite "*track etching*" connue pour la production de membrane polymère poreuse.

Dans son principe, cette technologie de "*track etching*" consiste à irradier la surface du polymère formant la paroi interne 11 par des ions lourds énergétiques.

Le bombardement par des ions lourds énergétiques provoque la formation de traces en dégradant localement la surface du polymère.

Les traces sont alors révélées sous la forme de perforations (ou pores) par une attaque chimique sélective.

On peut utiliser à titre d'exemple un bain chimique à base de soude pour créer les perforations dans l'épaisseur de la paroi interne 11.

On obtient ainsi des perforations réparties aléatoirement et uniformément dans la partie perforée 13a, 13b de la paroi interne 11.

A titre d'exemple non limitatif, on peut obtenir dans une paroi interne 11 en polycarbonate des perforations de taille sensiblement égale à 3,2 µm.

On obtient ainsi un logement 10 formé d'une double-paroi, se présentant globalement sous la forme d'un cylindre allongé ouvert à ses deux extrémités.

Afin d'obturer le logement, le dispositif intra-utérin comporte au moins un bouchon monté à une extrémité du logement 10.

Dans ce mode de réalisation, le dispositif intra-utérin comporte deux bouchons 20, 21 montés respectivement aux extrémités distale et proximale du logement 10.

Les bouchons 20, 21 peuvent être par exemple réalisés en titane.

Comme bien illustré à la figure 2, chaque bouchons 20, 21 est monté par friction à une extrémité de la paroi interne 11.

A cet effet, chaque bouchon 20, 21 comporte une première partie tronconique 20a, 21a dont le diamètre externe est, à sa petite base, légèrement inférieur au diamètre interne de la paroi interne 11. La première partie tronconique 20a, 21a s'élargit pour atteindre, à sa grande base, un diamètre externe légèrement supérieur au diamètre interne de la paroi interne 11.

Chaque première portion tronconique 20a, 21a est prolongée d'une seconde portion tronconique 20b, 21b s'élargissant à partir de la première portion tronconique 20a, 21a.

La seconde portion tronconique 20b, 21b est adaptée à être recouvert par une extrémité de la paroi externe 12 qui s'étend au-delà des extrémités de la paroi interne 11, jusqu'à un prolongateur du bouchon 20c, 21c.

Le diamètre le plus large de chaque bouchon 20, 21, et ici la grande base de la seconde partie tronconique 20b, 21b, a une dimension légèrement supérieure au diamètre interne de la paroi externe 12 de telle sorte que le montage du bouchon 20, 21 est réalisé par une légère déformation de la paroi externe 12 du logement, assurant une parfaite étanchéité du montage du bouchon.

Le bouchon distal 20 comporte en outre un premier prolongateur 20c en forme par exemple de tête de clou, permettant au médecin ou biologiste de manipuler le bouchon distal 20 pour permettre la fermeture ou l'ouverture du logement 10.

Le bouchon proximal 21 est prolongé par un second prolongateur 21c, formant une tige de fixation 21c adaptée à s'insérer en force dans un connecteur 30 (illustré partiellement aux figures 1 et 2).

Un tel connecteur est décrit notamment dans le document FR 2 903 879 A1 et n'a pas besoin d'être décrit ici en détail.

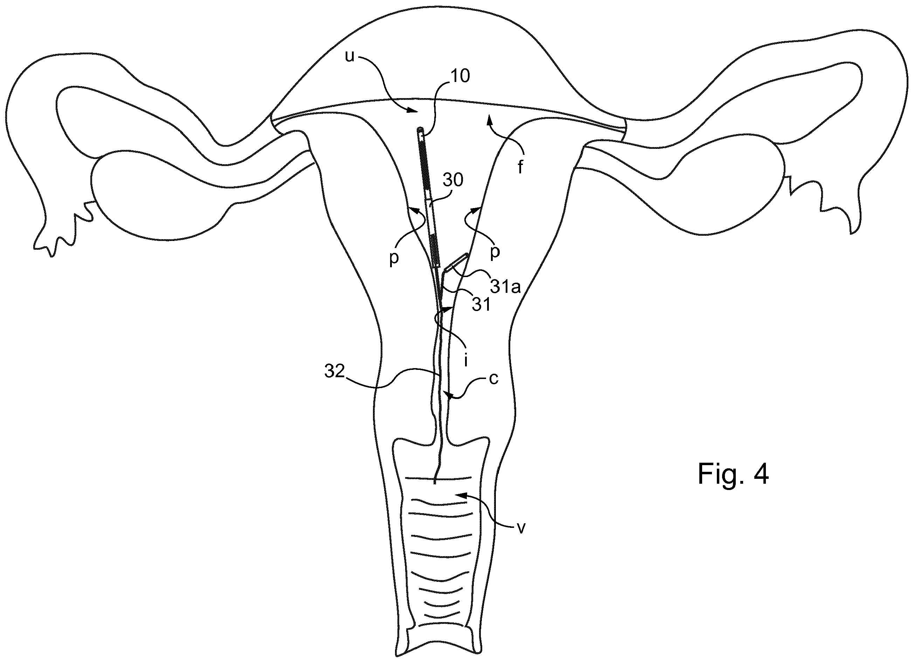

Seuls les éléments essentiels de ce connecteur sont rappelés ci-dessous en référence à la figure 4.

Un tel connecteur est adapté au montage d'un bras de stabilisation, utile pour le maintien en position du dispositif intra-utérin dans la matrice utérine, ainsi qu'au montage d'un fil de retrait.

Comme bien illustré à la figure 4, le connecteur 30 comprend un tube cylindrique s'étendant à l'extrémité proximale du logement 10.

Le tube cylindrique du connecteur 30 est par exemple en élastomère de silicone et est monté en force à son extrémité distale sur la tige de fixation 21c solidaire du bouchon proximal 21.

Un bras de stabilisation 31 est fixé à l'extrémité proximale du tube cylindrique du connecteur 30.

De manière générale, le bras de stabilisation 31 peut occuper une position repliée, s'étendant ainsi dans la direction longitudinale X du logement 10 et du connecteur 30, et une position déployée telle qu'illustrée à la figure 4 dans laquelle, par un effet ressort, le bras de stabilisation 31 se déplie, une extrémité 31a du bras venant en contact ponctuellement avec l'utérus u.

Le bras de stabilisation 31 peut être réalisé par exemple en acier et peut présenter à son extrémité 31a un manchon de protection en elastomère de silicone.

Par ailleurs, un fil de retrait en nylon 32 pour permettre le retrait du dispositif de l'utérus par traction est fixé à l'extrémité proximale du tube cylindrique du connecteur 30.

Le principe d'utilisation et de mise en place du dispositif intra-utérin est similaire à celui décrit dans le document FR 2 903 879 A1.

Comme bien illustré à la figure 4, le dispositif intra-utérin est introduit par les voies naturelles dans l'utérus au moyen d'un cathéter de transfert (non illustré).

Le cathéter de transfert est ensuite retiré permettant au bras de stabilisation 31 de se déployer et de maintenir le dispositif intra-utérin en place pour quelques heures ou quelques jours, permettant un développement préimplantatoire de l'embryon dans le milieu intra-utérin.

Grâce à la porosité de la paroi interne 11 du logement 10, au niveau des parties perforées 13a, 13b, les échanges entre les gamètes et/ou embryons contenus à l'intérieur du dispositif intra-utérin et les fluides utérins sont favorisés et permettent un développement optimal de l'embryon.

A cet effet, le dispositif intra-utérin est placé dans l'utérus u à proximité de la partie fundique f et l'extrémité 31a du bras de stabilisation 31 vient en contact uniquement avec une très faible portion de la paroi endométriale au niveau du canal isthmique i.

Ainsi, la couche endométriale qui recouvre la cavité utérine au niveau de la partie fundique f, la partie corporéale p et la partie du canal isthmique i sont très peu en contact avec le dispositif intra-utérin, évitant toute détérioration de l'endomètre qui pourrait être néfaste à la réimplantation ultérieure de l'embryon.

Par ailleurs, le fil de retrait 32 s'étend au travers du col c de l'utérus pour déboucher dans le vagin v et permettre une extraction aisée du dispositif par le praticien, par simple traction.

Bien entendu, l'invention n'est pas limitée aux exemples de réalisation décrits précédemment et de nombreuses modifications peuvent être apportées à ces exemples sans sortir du cadre de l'invention qui est définie par les revendications.

## Revendications

1. Dispositif intra-utérin récupérable comprenant un logement (10) adapté à contenir un ou plusieurs éléments choisis parmi le groupe comprenant un embryon, des gamètes mâles et/ou femelles, un ovocyte fécondé, un ovule non fécondé et une combinaison de ces éléments, ledit logement (10) comprenant une paroi interne (11) réalisée en un polymère biocompatible, ladite paroi interne (11) comprenant au moins une partie perforée (13a, 13b), et une paroi externe (12) recouvrant ladite paroi interne (11), la paroi externe (12) étant réalisée en un élastomère biocompatible et comprenant au moins une ouverture (14a, 14b), ledit dispositif intra-utérin étant **caractérisé en ce que** ladite au moins une partie perforée (13a, 13b) comprend des perforations de dimension comprises entre 1µm et 10µm, **en ce que** ledit élastomère biocompatible est un élastomère de silicone et **en ce que** ladite au moins une ouverture (14a, 14b) est disposée en vis-à-vis de ladite au moins une partie perforée (13a, 13b) de ladite paroi interne (11).

2. Dispositif conforme à la revendication 1, **caractérisé en ce que** lesdites perforations ont des dimensions comprises entre 2 µm et 8 µm.

3. Dispositif conforme à l'une des revendications 1 ou 2, **caractérisé en ce que** la paroi interne (11) est réalisée en polycarbonate.

4. Dispositif conforme à l'une des revendications 1 à 3, **caractérisé en ce que** ladite paroi interne (11) a au moins une partie perforée (13a, 13b) comprenant de 1 million à 5 millions de perforations par cm².

5. Dispositif conforme à l'une des revendications 1 à 4, **caractérisé en ce que** les perforations ont sensiblement une forme circulaire de diamètre compris entre 1 µm et 10 µm, et de préférence entre 2 µm et 8 µm.

6. Dispositif conforme à l'une des revendications 1 à 5, **caractérisé en ce que** le logement (10) est de forme allongée selon une direction longitudinale (X), ladite paroi externe (12) s'étendant au-delà des extrémités de ladite paroi interne (11) dans la direction longitudinale (X).

7. Dispositif conforme à la revendication 6, **caractérisé en ce qu'**il comporte au moins un bouchon (20, 21) monté à une extrémité du logement (10), ledit bouchon (20, 21) étant monté par friction à une desdites extrémités de la paroi interne (11) dudit logement (10) et étant partiellement recouvert par une extrémité de la paroi externe (12).

8. Dispositif conforme à l'une des revendications 1 à 7, **caractérisé en ce que** ledit logement (10) est de forme cylindrique allongée.

9. Dispositif conforme à la revendication 8, **caractérisé en ce que** la paroi externe (12) comprend au moins une ouverture (14a, 14b) s'étendant dans la direction longitudinale (X) du logement (10) et sur un secteur angulaire (a) du logement (10) compris entre 80° et 120°.

10. Dispositif conforme à l'une des revendications 8 ou 9, **caractérisé en ce que** la paroi interne (11) comprend deux parties perforées (13a, 13b), diamétralement opposées dans ladite forme cylindrique allongée du logement (10), et la paroi externe (12) comprend deux ouvertures (14a, 14b) en vis-à-vis respectivement desdites deux parties perforées (13a, 13b) de ladite paroi interne (11).

11. Dispositif conforme à l'une des revendications 1 à 10, **caractérisé en ce que** la paroi interne (11) et la paroi externe (12) sont réalisées en matériau transparent.

## Patentansprüche

1. Entnehmbare intrauterine Vorrichtung, enthaltend eine Aufnahmeausnehmung (10), die dazu geeignet ist, ein oder mehrere Elemente, ausgewählt aus der Gruppe umfassend einen Embryo, männliche und/oder weibliche Keimzellen, eine befruchtete Eizelle, eine nicht befruchtete Eizelle und eine Kombination dieser Elemente, zu beinhalten, wobei die Aufnahmeausnehmung (10) eine Innenwand (11) enthält, die aus einem biokompatiblen Polymer hergestellt ist, wobei die Innenwand (11) zumindest einen perforierten Abschnitt (13a, 13b) aufweist, sowie eine Außenwand (12), welche die Innenwand (11) überdeckt, wobei die Außenwand (12) aus einem biokompatiblen Elastomer hergestellt ist und zumindest eine Öffnung (14a, 14b) aufweist, wobei die intrauterine Vorrichtung **dadurch gekennzeichnet ist, dass** der zumindest eine perforierte Abschnitt (13a, 13b) Perforationen mit Abmessungen zwischen 1 µm und 10 µm aufweist, dass das biokompatible Elastomer ein Silikonelastomer ist und dass die zumindest eine Öffnung (14a, 14b) dem zumindest einen perforierten Abschnitt (13a, 13b) der Innenwand (11) gegenüberliegend angeordnet ist.

2. Vorrichtung nach Anspruch 1, **dadurch gekennzeichnet, dass** die Perforationen Abmessungen zwischen 2 µm und 8 µm haben.

3. Vorrichtung nach einem der Ansprüche 1 oder 2, **dadurch gekennzeichnet, dass** die Innenwand (11) aus Polycarbonat hergestellt ist.

4. Vorrichtung nach einem der Ansprüche 1 bis 3, **dadurch gekennzeichnet, dass** die Innenwand (11) zumindest einen perforierten Abschnitt (13a, 13b) mit 1 Million bis 5 Millionen Perforationen pro cm² aufweist.

5. Vorrichtung nach einem der Ansprüche 1 bis 4, **dadurch gekennzeichnet, dass** die Perforationen im Wesentlichen eine kreisrunde Form mit einem Durchmesser zwischen 1 µm und 10 µm und vorzugsweise zwischen 2 µm und 8 µm haben.

6. Vorrichtung nach einem der Ansprüche 1 bis 5, **dadurch gekennzeichnet, dass** die Aufnahmeausnehmung (10) eine in einer Längsrichtung (X) langgestreckte Form aufweist, wobei die Außenwand (12) sich in der Längsrichtung (X) über die Enden der Innenwand (11) hinaus erstreckt.

7. Vorrichtung nach Anspruch 6, **dadurch gekennzeichnet, dass** sie zumindest einen Stopfen (20, 21) enthält, der an einem Ende der Aufnahmeausnehmung (10) gelagert ist, wobei der Stopfen (20, 21) durch Reibschluss an einem der Enden der Innenwand (11) der Aufnahmeausnehmung (10) gelagert ist und von einem Ende der Außenwand (12) teilweise überdeckt wird.

8. Vorrichtung nach einem der Ansprüche 1 bis 7, **dadurch gekennzeichnet, dass** die Aufnahmeausnehmung (10) eine langgestreckte Zylinderform hat.

9. Vorrichtung nach Anspruch 8, **dadurch gekennzeichnet, dass** die Außenwand (12) zumindest eine Öffnung (14a, 14b) aufweist, die sich in Längsrichtung (X) der Aufnahmeausnehmung (10) und über einen Winkelsektor (α) der Aufnahmeausnehmung (10) zwischen 80° und 120° erstreckt.

10. Vorrichtung nach einem der Ansprüche 8 oder 9, **dadurch gekennzeichnet, dass** die Innenwand (11) zwei diametral entgegengesetzte perforierte Abschnitte (13a, 13b) in der langgestreckten Zylinderform der Aufnahmeausnehmung (10) enthält und die Außenwand (12) zwei Öffnungen (14a, 14b) aufweist, die jeweils den beiden jeweiligen perforierten Abschnitten (13a, 13b) der Innenwand (11) gegenüberliegen.

11. Vorrichtung nach einem der Ansprüche 1 bis 10, **dadurch gekennzeichnet, dass** die Innenwand (11) und die Außenwand (12) aus einem transparenten Material hergestellt sind.

## Claims

1. A recoverable intra-uterine device comprising a housing (10) configured to contain one or more items chosen from the group comprising an embryo, male and/or female gametes, a fertilized oocyte, an unfertilized ovum and a combination of these items, said housing (10) comprising an inside wall (11) formed from a biocompatible polymer, said inside wall (11) comprising at least one perforated part (13a, 13b), and an outside wall (12) covering said inside wall (11), the outside wall (12) being formed from a biocompatible elastomer and comprising at least one opening (14a, 14b), said intra-uterine device being **characterized in that** said at least one perforated part (13a, 13b) comprises perforations of dimensions comprised between 1 µm and 10 µm, **in that** said biocompatible elastomer is a silicone elastomer and **in that** said at least one opening (14a, 14b) is disposed facing said at least one perforated part (13a, 13b) of said inside wall (11).

2. A device according to claim 1, **characterized in that** said perforations have dimensions comprised between 2 µm and 8 µm.

3. A device according to one of claims 1 or 2, **characterized in that** the inside wall (11) is formed from polycarbonate.

4. A device according to one of claims 1 to 3, **characterized in that** said inside wall (11) has at least one perforated part (13a, 13b) comprising from 1 million to 5 million perforations per cm².

5. A device according to one of claims 1 to 4, **characterized in that** the perforations are of substantially circular shape of diameter comprised between 1 µm and 10 µm, and preferably between 2 µm and 8 µm.

6. A device according to one of claims 1 to 5, **characterized in that** the housing (10) has a shape which is elongate in a longitudinal direction (X), said outside wall (12) extending beyond the ends of said inside wall (11) in the longitudinal direction (X).

7. A device according to claim 6, **characterized in that** it comprises at least one plug (20, 21) mounted at an end of the housing (10), said plug (20, 21) being mounted by friction at one of said ends of the inside wall (11) of said housing (10) and being partially covered by an end of the outside wall (12).

8. A device according to one of claims 1 to 7, **characterized in that** said housing (10) is of elongate cylindrical shape.

9. A device according to claim 8, **characterized in that** the outside wall (12) comprises at least one opening (14a, 14b) extending in the longitudinal direction (X) of the housing (10) and over an angular sector (a) of the housing (10) comprised between 80° and 120°.

10. A device according to one of claims 8 or 9, **characterized in that** the inside wall (11) comprises two perforated parts (13a, 13b), which are diametrically opposite in said elongate cylindrical shape of the housing (10), and the outside wall (12) comprises two openings (14a, 14b) respectively facing said two perforated parts (13a, 13b) of said inside wall (11).

11. A device according to one of claims 1 to 10, **characterized in that** the inside wall (11) and the outside wall (12) are formed from transparent material.
